# EUROPEAN PATENT SPECIFICATION

(11) **EP 3 384 014 B1**
(45) Date of publication and mention of the grant of the patent: **09.12.2020**
(21) Application number: 16781798.0
(22) Date of filing: 14.10.2016
(51) Int. Cl.: C12N 5/079

(54) **METHOD FOR PREPARING CORNEAL TISSUE**
VERFAHREN ZUR HERSTELLUNG VON HORNHAUTMATERIAL
PROCÉDÉ DE PRÉPARATION DE TISSU CORNÉEN

(30) Priority: 30.11.2015 DE 102015120716
(43) Date of publication of application: 10.10.2018
(73) Proprietor: Biotronik AG, 8180 Bülach (CH)
(72) Inventor: RZANY, Alexander, 90449 Nürnberg (DE); ERDBRÜGGER, Wilhelm, 78467 Konstanz (DE)
(74) Representative: Randoll, Sören
(86) International application number: PCT/EP2016/074716
(87) International publication number: WO 2017/092918

(56) References cited:
- CN-A- 103 908 700
- PONCE MARQUEZ S ET AL: "Decellularization of bovine corneas for tissue engineering applications", ACTA BIOMATERIALIA, ELSEVIER, AMSTERDAM, NL, vol. 5, no. 6, 1 July 2009 (2009-07-01), pages 1839-1847, XP026161748, ISSN: 1742-7061, DOI: 10.1016/J.ACTBIO.2009.02.011 [retrieved on 2009-02-11]
- OH JOO YOUN ET AL: "Processing Porcine Cornea for Biomedical Applications", TISSUE ENGINEERING PART C-METHODS, vol. 15, no. 4, December 2009 (2009-12), pages 635-645, XP002764210,
- SHAFIQ MARYAM A ET AL: "Decellularized Human Cornea for Reconstructing the Corneal Epithelium and Anterior Stroma", TISSUE ENGINEERING PART C-METHODS, vol. 18, no. 5, May 2012 (2012-05), pages 340-348, XP002764211,
- LYNCH AMY P ET AL: "Strategies for developing decellularized corneal scaffolds", EXPERIMENTAL EYE RESEARCH, ACADEMIC PRESS LTD, LONDON, vol. 108, 31 December 2012 (2012-12-31), pages 42-47, XP028980401, ISSN: 0014-4835, DOI: 10.1016/J.EXER.2012.12.012
- WILSON SAMANTHA L ET AL: "Keeping an eye on decellularized corneas: a review of methods, characterization and applications.", JOURNAL OF FUNCTIONAL BIOMATERIALS 2013, vol. 4, no. 3, 2013, pages 114-161, XP002764212, ISSN: 2079-4983

## Description

The present invention relates to a method for preparing corneal tissue for applications predominantly in transplantation, and to the use of a solution for decellularizing corneal tissue. The present invention further relates to transparent corneal tissue.

The decellularization of tissue is used to prevent immune responses, which may occur when exogenous tissue is detected by the immune system. To mitigate or to prevent such responses, cell membranes, intracellular proteins, cell nuclei and other cell components are removed as completely as possible from the tissue during decellularization, so as to yield a practically pure extracellular matrix. Cells and cell components remaining in the tissue would represent in particular potent starting points for an undesirable immune response, which in other tissue may result in the calcification of the biological implant material, for example.

In addition, the decellularization of corneal tissue is subject to special requirements. Since the cornea is located in the outer region of where light passes through the eyeball and light shines through it for people to see, it is essential for the recipient of the implant and for the vision of this person that the implant not only does not cause any immune responses, but also is transparent and clear.

It is furthermore important when decellularizing corneal tissue that the decellularization step is carried out gently enough for the structure of the extracellular matrix to remain substantially unaffected, while removing all cells and cell components contained therein from the tissue to the greatest extent possible. In this way, the chemical, altering intervention into the tissue can be kept as low as possible, while preventing immune responses at the same time.

CN 103 908 700 A relates to a decellularization cornea preparation method. Acta Biomaterialia 5, 2009, 1839 discloses the decellularization of bovine corneas for tissue engineering applications. Tissue Engineering Part C, Volume 15, 4, 2009, 635 discloses processing porcine cornea for biomedical applications. Tissue Engineering Part C, Volume 18, 5, 2012, 340 discloses decellularized human cornea for reconstructing the corneal epithelium and anterior stroma.

It is therefore desirable to decellularize corneal tissue very gently, so that the tissue structure is altered little, and transparency is preserved.

Against this background, a method for preparing corneal tissue is proposed, which allows transparent corneal tissue to be provided, which at its best does not trigger any immune responses.

In particular, after the corneal tissue is provided, a method for preparing corneal tissue is proposed, comprising the following steps:
decellularizing the tissue by way of a detergent that is gentle on the tissue in the presence of a polyalcohol.

Xenogenous tissue, and under some circumstances also human donor issue, must be thoroughly cleansed and prepared prior to implantation. The tissue is modified, to the greatest extent possible, in such a way that it is not identified as foreign tissue by the body and has as long a life span as possible. As was already noted, it is additionally necessary in the case of corneal tissue for the tissue to have optical transparency to allow the patient to have adequate vision following the implantation. Such corneal tissue can be provided by the method proposed herein.

Detergents that are gentle on the tissue shall be understood herein to mean decellularizing agents that fulfill the task of removing cell membranes, intracellular proteins, cell nuclei and other cell components as completely as possible from the tissue, without changing the remaining tissue structure, or changing this only little; in other words, the structure in the intracellular matrix remains substantially unaffected. For example, in the case of decellularization using a gentle decellularizing agent, the shrinkage temperature also remains essentially unchanged, compared to native tissue. Strong and at times highly concentrated decellularizing agents such as sodium dodecyl sulfate (SDS), in contrast, considerably influence the extracellular structure, altering this significantly. Such an alteration is undesirable, especially in the case of the cornea, since, without being bound to this theory, it is assumed by the inventors that clouding of the corneal tissue, which makes it impossible to see through the tissue, is caused by alterations in the collagen fibrils structure.

However, surprisingly it was found that it is not sufficient to simply use a detergent that is gentle on the tissue, as described herein, when decellularizing corneal tissue. Tissue that is decellularized under customary conditions by way of a decellularizing agent that is gentle on the tissue exhibits strong clouding. It was found according to the invention that the decellularization of corneal tissue must also be carried out in the presence of a polyalcohol to yield transparent tissue.

Suitable polyalcohols are selected from the group comprising or consisting of glycerol, 1,2-propanediol, 1,3-propanediol, polyethylene glycol, polypropylene glycol, ethylene glycol, 1,2-butanediol, 1,3-butanediol, 1,4-butanediol, 2,3-butanediol, 1,2,3-butanetriol, 1,2,4-butanetriol, 1,2-pentanediol, 1,3-pentanediol, 1,4-pentanediol, 1,5-pentanediol, 2,3-pentanediol, 2,4-pentanediol, 1,2,5-pentanetriol, 1,3,4-pentanetriol, 1,3,5-pentanetriol, 1,2,3,4-butanetetrol, preferably meso-1,2,3,4-butanetetrol, and further alditols including a linear C5 to C8 carbon chain. Polyethylene glycols preferably have an average molecular weight between 100 g/mol and 4000 g/mol, and in particular 400 g/mol. In a preferred embodiment, the polyalcohol is selected from the group comprising or consisting of glycerol, polyethylene glycol and ethylene glycol. In a further preferred embodiment, the polyalcohol is glycerol.

The polyalcohols proposed herein are preferably introduced in the form of aqueous solutions. Within the scope of the present application, the information % v/v or vol.% refers to a volume percent fraction. Unless indicated otherwise with respect to a solution, the solvent used for the solutions herein is water. 100 ml solution comprising 5% v/v glutaraldehyde accordingly comprises 5 ml glutaraldehyde. Within the scope of the present application, the information % w/v or wt.% refers to a percent by weight fraction. 100 ml solution comprising 0.9% w/v sodium chloride accordingly comprises 0.9 g sodium chloride.

In one embodiment of the method proposed herein, the decellularization is carried out in the presence of a polyalcohol, which is present in the form of a 10 to 70% solution, and preferably a 30 to 50% solution. Without being bound to this theory, the inventors assume that introducing the polyalcohol, preferably in the above-mentioned concentration, results in osmotic pressure as a result of the difference in the concentration of the polyalcohol in the tissue and in the surrounding solution, causing the polyalcohol to be introduced into the corneal tissue, releasing water. It is further assumed that this favorably impacts the preservation of the extracellular matrix during the decellularization. In contrast, it is assumed that a high concentration of water in the corneal tissue during the decellularization results in an alteration of the collagen fibrils, causing clouding of the tissue.

According to the invention, a detergent that is gentle on the tissue, serving as the decellularizing agent, can be provided by using lipopeptides, and in particular cyclic and/or amphiphilic lipopeptides. In particular β-hydroxy fatty acid-containing or β-amino-fatty acid-containing peptides may be used here. It has been shown that the lipopeptides, as described herein, show outstanding results in the decellularization of corneal tissue. Using this class of substances, cell components can be removed very gently from the tissue. The structure of the extracellular matrix is preserved very well when a lipopeptide is used. For example, measurements of the shrinkage temperature show that this remains in the range of native tissue even after the treatment with a lipopeptide.

Particularly preferably, the detergent for decellularization includes a cyclic lipoheptapeptide, and in particular surfactin. In this preferred embodiment of the invention, a cyclic lipoheptapeptide, and in particular a detergent comprising surfactin, is used for decellularization. In particular, the detergent comprises a cyclic lipoheptapeptide having a cyclic structure as shown hereafter:

(CH₃)₂-CH-(CH₂)ₙ → CH-CH₂-CO → L-Glu → L-Leu → D-Leu → L-Val → L-Asp → D-Leu → L-Leu → O

For applications for the present invention, n may be 8 to 12 and Glu, Leu, Val, Asp, wherein Glu, Leu, Val, Asp denote the amino acids glutamic acid, leucine, valine, and aspartic acid, respectively.

It is likewise advantageous to use lipopeptides such as daptomycin, caspofungin, arthrofactin, echinocandins, iturins, syringomycins, syringopeptides and/or polymyxins. Advantageously, the detergent comprises at least one lipopeptide selected from the following list: surfactin, daptomycin, caspofungin, arthrofactin, or the group of the echinocandins, iturins, syringomycins, syringopeptides and polymyxins.

In a further embodiment of the method proposed herein, the decellularization is carried out in the presence of a second decellularizing agent, preferably deoxycholic acid. Deoxycholic acid acts as a second, less concentrated decellularizing agent and, in combination with the detergent described herein, results in a more comprehensive decellularization, while once again, due to the presence of a polyalcohol, the tissue structure, is not altered so much that clouding of the corneal tissue occurs. It was found to be particularly advantageous that the use of a second decellularizing agent, in combination with the detergent described herein, also causes the nuclear membranes to be dissolved, whereby better cell disruption is achieved. As an alternative to deoxycholic acid, it is also possible, for example, to use Triton-X 100 or Tween 20. The second decellularizing agent is preferably added in a very low concentration, preferably in a concentration of 0.1 to 1 wt.%.

In a further embodiment, the method proposed herein includes a step of treating the decellularized tissue with at least one α--galactosidase in the presence of a polyalcohol. The polyalcohol is preferably the same as that used in the decellularization step.

α-galactosidases, or else α-D-galactoside galactohydrolase, E.C. 3.2.1.22, are enzymes able to catalyze the hydrolysis of galactosyl residues of the non-reducing ends of a plurality of oligosaccharides and polysaccharides, and of galactolipids and glycoproteins.

The additional use of an α-galactosidase has the advantage that naturally occurring xenogeous galactose-α-1,3-galactose-β-1,4-*N*-acetylglucosamine epitopes (α-1,3-galactosyl residues, α-Gal epitopes) of the corneal tissue can be effectively removed. Removal of the α-Gal epitopes is desirable since these can result in strong immune responses, forming antibodies. The concentration of α-Gal epitopes on the surface of the tissue can be decreased by way of decellularization; however, this takes place under rather harsh decellularization conditions, which frequently also result in alteration of the extracellular matrix.

In a preferred embodiment, the corneal tissue is treated with one α-galactosidase type. However, it is also possible to use a combination of α-galactosidases. This means that the α-galactosidases used in combination have a different structure and/or origin, which means that the α-galactosidases were produced in different living beings and/or have different structures. α-galactosidases can diverge significantly in terms of the task thereof, structurally, and in the action thereof, depending on the origin. This is closely related to the fact that a plurality of organisms produce α-galactosidases, such as Archaea, bacteria, fungi, plants or animals. One possible cause for the diversity of α-galactosidases may lie in the task of the particular organism. For example, a grouping may be result of the pH dependence of the enzymatic activity.

In a further embodiment, the use of alkaline α-galactosidases is proposed for the method proposed herein. Alkaline α-galactosidases are characterized by having a high, or the highest, enzymatic activity in an alkaline medium (at times also in a slightly acidic environment) and furthermore having a high substrate specificity. The use of alkaline α-galactosidases is advantageous since it is thus made possible to efficiently use DNases and RNases as well, in addition to the alkaline α-galactosidases. DNases and RNases are used to remove any remaining ribonucleic acids from the tissue. By combining α-galactosidases with DNases and/or RNases, further improved protection against immune responses and calcification must thus be achieved. In particular, preferred α-galactosidases are those that show the greatest specific enzymatic activity in a slightly alkaline or at least not strongly acidic range, in particular in a pH range of 5.5 to 8.0, more preferably in a pH range of 6.0 to 7.8, and most preferably in a pH range of 7.0 to 7.6, since in this pH range a combination with DNases and RNases is possible in such a way that the enzymes used can work efficiently. Alkaline α-galactosidases from Arabidopsis thaliana, Cucumis melo, Cucumis sativus, Oryza sativa, such as the Japonica group, Pisum sativum, Solanum Lycopersicum, Tetragonia tetragonioides, and Zea mays are preferred.

In a preferred embodiment, the method proposed herein is carried out in the presence of at least one α-galactosidase from Arabidopsis thaliana, Cucumis melo, Cucumis sativus, Oryza sativa, such as the Japonica group, Pisum sativum, Solanum Lycopersicum, Tetragonia tetragonioides, Zea mays or green coffee beans. In a further preferred embodiment, the α-galactosidase is from green coffee beans or from Cucumis melo.

In a further embodiment, the method proposed herein includes a step of treating the decellularized corneal tissue with a DNase in the presence of a polyalcohol. In a particularly preferred embodiment, the method proposed herein includes a step of treating the decellularized tissue with a DNase and an α--galactosidase in the presence of a polyalcohol as described herein. The step of treating the decellularized tissue with a DNase and/or an α-galactosidase in the presence of a polyalcohol is preferably carried out in a buffer solution, advantageously at a pH of 5.5 to 8.0. For this purpose, Dulbecco's phosphate-buffered saline (DPBS) without calcium and magnesium, and tris(hydroxymethyl)aminomethane (TRIS) or 2-[4-(2-hydroxyethyl-1-piperazinyl]ethanesulfonic acid (HEPES) are suitable buffers.

Advantageously, the corneal tissue is rinsed at least once, and preferably several times, with a suitable solvent, and in particular a saline solution and/or an alcohol solution, prior to and particularly preferably after the decellularization, wherein these rinsing solutions contain a polyalcohol proposed herein. These solutions may also include a suitable buffer, such as DPBS. In particular, these solutions comprise glycerol. Particularly advantageous are physiological sodium chloride solutions (corresponds to a 0.9% w/v NaCl solution; 100 ml solution comprising 0.9% w/v sodium chloride accordingly comprises 0.9 g sodium chloride) containing a polyalcohol proposed herein.

In a preferred embodiment, after corneal tissue is provided, a method for preparing corneal tissue is proposed, comprising the following steps:
- decellularizing the tissue by way of a detergent that is gentle on the tissue in the presence of a polyalcohol proposed herein, wherein the decellularization is carried out in the presence of a second decellularizing agent;
- treating the decellularized tissue with a DNase in the presence of a polyalcohol; and
- treating the decellularized tissue with α-galactosidase in the presence of a polyalcohol.

According to a further aspect of the present invention, the use of a solution is proposed which comprises at least one gentle decellularizing agent, preferably a cyclic lipopeptide, more preferably a lipopeptide having amphiphilic properties, composed of a hydrophilic basic structure and a hydrophobic side chain, and most preferably surfactin, and a polyalcohol, preferably glycerol, as the detergents for decellularizing corneal tissue.

The above-described use is further proposed with a solution comprising at least surfactin, daptomycin, caspofungin, arthrofactin, an echinocandin, an iturin, a syringomycin, a syringopeptide and/or a polymyxin, and a polyalcohol as described herein.

According to a further aspect of the present invention, corneal tissue is proposed which was treated by way of a method described herein and is thus obtainable.

In a particular embodiment, furthermore transparent decellularized corneal tissue is proposed, which comprises a polyalcohol, and in particular glycerol. Furthermore, transparent, decellularized corneal tissue is proposed, which comprises a polyalcohol, and in particular glycerol, and which on the surface is furthermore free of, or approximately free of, α-Gal epitopes and/or DNA.

Corneal tissue can have different origins for the invention described herein, and may be of human or of animal origin. In the case of animal tissue, the cornea may be of pig, sheep, goat, horse, crocodile, kangaroo, ostrich, monkey, preferably primate, octopus, rabbit and bovine origin.

The invention shall be described in more detail hereafter based on exemplary embodiments according to the invention and compared to samples not treated according to the invention. Samples of the original DNA content prior to the tissue preparation and after the tissue preparation according to a method proposed herein were compared. The DNA content forms a direct measure of the removal of cellular components from the biological tissue. A tissue preparation according to the method proposed herein, carried out by way of example with surfactin, deoxycholic acid and a DNase here, results in a removal of DNA from the corneal tissue.
Corneal tissue that was decellularized by a method according to the invention in the presence of a polyalcohol proposed herein. The corneal samples have an extremely high degree of transparency, which allows printed manner on the surface beneath the cornea sample to be identified without difficulty and to be read.

Corneal tissue that was decellularized by a method not according to the invention in the absence of a polyalcohol proposed herein. The tissue is very cloudy and so opaque that printed matter on the surface beneath the cornea sample cannot even vaguely be identified.

It was found that the decellularization proposed herein can be used not only to produce transparent tissue, but also to achieve a significant reduction, or almost complete removal, of α-Gal epitopes, whereby the likelihood of an immune response to transplanted cornea is minimized.

### Example 1

Lenticels (which is to say pieces of corneal tissue) are stored in cell culture medium, for example comprising or consisting of DMEM ((Dulbecco's Modified Eagle's Medium) or similarly acting solutions.
Afterwards, the cell culture medium is removed, and the lenticels are rinsed 3 times with 5 ml physiological NaCl solution comprising 40 wt.% glycerol at room temperature and under slight movement.

Thereafter, the samples are decellularized by treating them with the following solution for approximately 20 hours at 37°C: 0.06 wt.% surfactin, 0.5 wt.% sodium deoxycholate and 40 wt.% glycerol in physiological NaCl.
The samples are rinsed 5 times with 5 ml NaCl plus 40 wt.% glycerol at room temperature and under slight movement and then stored in the refrigerator.

### Example 2

In one refinement of the present invention, samples from Example 1 are cleaned further enzymatically in a further treatment step. For this purpose, the lenticels are treated for 16 hours at 37°C with 40 U/ml DNase I and 0.1 U/ml green coffee bean (GCB) α-galactosidase in 50 mM HEPES (pH 6.0), 2 mM CaCl, 5 mM MgCl comprising 40 wt.% glycerol.

The samples are subsequently rinsed 5 times with 5 ml NaCl comprising 40 wt.% glycerol at room temperature and under slight movement.

For the determination of the DNA content of the samples, these are enzymatically treated with 15 U proteinase K in DPBS and finally measured.

For the determination of the remaining α-Gal epitopes on the surface of the samples, these were determined with the aid of an M86 antibody-based immuno assay.

### Comparative example

Lenticels are stored in a cell culture medium. The samples are divided. Individual samples are treated with 15 U/ml proteinase K in DPBS for analytical purposes and then subjected to a DNA determination.
Afterwards, the storage solution is removed from further samples, and the samples are rinsed 3 times with 5 ml physiological NaCl solution. Thereafter, the samples are decellularized for approximately 20 hours with 5 ml 0.06 wt.% surfactin and 0.5 wt.% deoxycholic acid. The comparative example was thus carried out in the absence of a polyalcohol as described herein.

For further analysis, the samples are treated with 15 U/ml proteinase K in PBS, rinsed 5 times with 8 ml NaCl, and furthermore treated for 16 hours with 40 U/ml DNase I.

The DNA content in the untreated lenticels was determined to be 2.42 +/- 0.38 µg/cornea. Treated lenticels, in contrast, no longer contained any measurable DNA.

The exemplary embodiments described here are intended to illustrate the invention. The number and/or design of the rinsing steps (in particular the concentrations and composition of the solution for rinsing or of the buffer solution) may be varied by a person skilled in the art.

## Claims

1. A method for preparing corneal tissue, comprising the following steps:
• decellularizing the tissue in the presence of a polyalcohol by way of a detergent that is so gentle on the tissue that the structure in the intracellular matrix remains substantially unaffected.

2. The method according to claim 1, **characterized in that** the detergent for decellularization comprises a cyclic lipopeptide.

3. The method according to claim 1 or 2, characterized the detergent comprises surfactin, daptomycin, caspofungin, arthrofactin, an echinocandin, an iturin, a syringomycin, a syringopeptide and/or a polymyxin.

4. The method according to any one of the preceding claims, **characterized in that** the decellularization is carried out in the presence of a second decellularizing agent.

5. The method according to any one of the preceding claims, **characterized by** comprising the following step:
• treating the decellularized tissue with a DNase in the presence of a polyalcohol.

6. The method according to any one of the preceding claims, **characterized by** comprising the following step:
• treating the decellularized tissue with α-galactosidase in the presence of a polyalcohol.

7. The method according to claim 6, **characterized in that** the α-galactosidase is from green coffee beans or from Cucumis melo.

8. The method according to any one of the preceding claims, **characterized in that** the polyalcohol is selected from the group comprising or consisting of glycerol, 1,2-propanediol, 1,3-propanediol, polyethylene glycol, polypropylene glycol, ethylene glycol, 1,2-butanediol, 1,3-butanediol, 1,4-butanediol, 2,3-butanediol, 1,2,3-butanetriol, 1,2,4-butanetriol, 1,2-pentanediol, 1,3-pentanediol, 1,4-pentanediol, 1,5-pentanediol, 2,3-pentanediol, 2,4-pentanediol, 1,2,5-pentanetriol, 1,3,4-pentanetriol, 1,3,5-pentanetriol, 1,2,3,4-butanetetrol, preferably meso-1,2,3,4-butanetetrol, and further alditols including a linear C5 to C8 carbon chain.

9. Use of a solution comprising at least one cyclic lipopeptide and a polyalcohol as detergents for decellularizing corneal tissue.

10. The use according to claim 9, **characterized in that** the solution comprises glycerol as the polyalcohol.

## Patentansprüche

1. Verfahren zur Aufbereitung von Corneagewebe umfassend die folgenden Schritte:
• Dezellularisierung des Gewebes in Gegenwart eines Polyalkohols mittels eines so gewebeschonenden Detergens, dass die Struktur in der extrazellulären Matrix möglichst unbeeinflusst bleibt.

2. Verfahren nach Anspruch 1, **dadurch gekennzeichnet, dass** das Detergens zur Dezellularisierung ein zyklisches Lipopeptid enthält.

3. Verfahren nach Anspruch 1 oder 2, **dadurch gekennzeichnet dass** das Detergens Surfactin, Daptomycin, Caspofungin, Arthrofactin, ein Echinocandin, ein Iturin, ein Syringomycin, ein Syringopeptid und/oder ein Polymyxin enthält.

4. Verfahren nach einem der vorangegangenen Ansprüche, **dadurch gekennzeichnet, dass** die Dezellularisierung in Gegenwart von einem zweiten Dezellularisierungsmittel durchgeführt wird.

5. Verfahren nach einem der vorangegangenen Ansprüche, **dadurch gekennzeichnet, dass** das Verfahren folgenden Schritt umfasst:
• Behandeln des dezellularisierten Gewebes mit einer DNAse in Gegenwart eines Polyalkohols.

6. Verfahren nach einem der vorangegangenen Ansprüche, **dadurch gekennzeichnet, dass** das Verfahren folgenden Schritt umfasst:
• Behandeln des dezellularisierten Gewebes mit α-Galactosidase in Gegenwart eines Polyalkohols.

7. Verfahren nach Anspruch 6, **dadurch gekennzeichnet, dass** die α-Galactosidase von der grünen Kaffeebohne oder von Cucumis melo stammt.

8. Verfahren nach einem der vorangegangenen Ansprüche, **dadurch gekennzeichnet, dass** der Polyalkohol ausgesucht ist aus der Gruppe umfassend oder bestehend aus Glycerin, 1,2-Propandiol, 1,3-Propandiol, Polyethylenglykol, Polypropylenglykol, Ethylenglykol, 1,2-Butandiol, 1,3-Butandiol, 1,4-Butandiol, 2,3-Butandiol, 1,2,3-Butantriol, 1,2,4-Butantriol, 1,2-Pentandiol, 1,3-Pentandiol, 1,4-Pentandiol, 1,5-Pentandiol, 2,3-Pentandiol, 2,4-Pentandiol, 1,2,5-Pentantriol, 1,3,4-Pentantriol, 1,3,5-Pentantriol, 1,2,3,4-Butantetrol, bevorzugt meso-1,2,3,4-Butantetrol, und weitere Alditole mit einer linearen C5 bis C8 Kohlenstoffkette.

9. Verwendung einer Lösung, die mindestens ein zyklisches Lipopeptid und einen Polyalkohol enthält, als Detergens zur Dezellularisierung von Corneagewebe.

10. Verwendung nach Anspruch 9, **dadurch gekennzeichnet, dass** die Lösung Glycerin als Polyalkohol enthält.

## Revendications

1. Procédé de préparation de tissu cornéen comprenant les étapes suivantes :
• la décellularisation du tissu en présence d'un polyalcool au moyen d'un détergent qui est si doux sur le tissu que la structure dans la matrice intracellulaire reste essentiellement non affectée

2. Procédé selon la revendication 1, **caractérisé en ce que** le détergent pour la décellularisation comprend un lipopeptide cyclique.

3. Procédé selon la revendication 1 ou la revendication 2, caractérisé le détergent comprend une surfactine, une daptomycine, une caspofungine, une arthrofactine, une échinocandine, une iturine, une syringomycine, un syringopeptide et/ou une polymyxine.

4. Procédé selon l'une quelconque des revendications précédentes, **caractérisé en ce que** la décellularisation est effectuée en présence d'un deuxième agent de décellularisation.

5. Procédé selon l'une quelconque des revendications précédentes, caractérisé comme comprenant l'étape suivante :
• le traitement du tissu décellularisé avec une ADNase en présence d'un polyalcool.

6. Procédé selon l'une quelconque des revendications précédentes, caractérisé comme comprenant l'étape suivante :
• le traitement du tissu décellularisé avec une α-galactosidase en présence d'un polyalcool.

7. Procédé selon la revendication 6, **caractérisé en ce que** l'a-galactosidase provient de graines de café vert ou du melon.

8. Procédé selon l'une quelconque des revendications précédentes, **caractérisé en ce que** le polyalcool est choisi dans le groupe comprenant ou constitué du glycérol, du 1,2-propane diol, du 1,3-propane diol, du polyéthylène glycol, du polypropylène glycol, de l'éthylène glycol, du 1,2-butane diol, du 1,3-butane diol, du 1,4-butane diol, du 2,3-butane diol, du 1,2,3-butane triol, du 1,2,4-butane triol, du 1,2-pentane diol, du 1,3-pentane diol, du 1,4-pentane diol, du 1,5-pentane diol, du 2,3-pentane diol, du 2,4-pentane diol, du 1,2,5-pentane triol, du 1,3,4-pentane triol, du 1,3,5-pentane triol, du 1,2,3,4-butane tétrol, de préférence du méso-1,2,3,4-butane tétrol et d'autres alditols comportant une chaîne carbonée en C5 à C8 linéaire.

9. Utilisation d'une solution comprenant au moins un lipopeptide cyclique et un polyalcool en tant que détergents pour décellulariser du tissu cornéen.

10. Utilisation selon la revendication 9, **caractérisée en ce que** la solution comprend du glycérol en tant que polyalcool.
